Europäisches Patentamt

(19) European Patent Office

Office européen des brevets

(11) Publication number : **0 503 829 A2**

# (12) EUROPEAN PATENT APPLICATION

(21) Application number : **92301844.4**

(22) Date of filing : **04.03.92**

(51) Int. Cl.[5] : **A61K 37/64**

(30) Priority : **08.03.91 US 666540**

(43) Date of publication of application :
**16.09.92 Bulletin 92/38**

(84) Designated Contracting States :
**AT BE CH DE DK FR GB IT LI LU NL PT SE**

(71) Applicant : **CIBA-GEIGY AG**
**Klybeckstrasse 141**
**CH-4002 Basel (CH)**

(71) Applicant : **THE BOARD OF REGENTS THE**
**UNIVERSITY OF TEXAS SYSTEM**
**6723 Bertner Avenue**
**Houston, Texas 77030 (US)**

(71) Applicant : **UCP GEN-PHARMA AG**
**Solothurnstrasse 24**
**CH-3422 Kirchberg (CH)**

(72) Inventor : **Wallis, Robert Brian**
**Leechpends Hill, Lower Beeding**
**Horsham, W. Sussex (GB)**
Inventor : **Fidler, Isaiah J.**
**Kingwood**
**TX 77345 (US)**
Inventor : **Esumi, Noriko**
**Houston**
**TX 77054 (US)**

(74) Representative : **Sharman, Thomas et al**
**CIBA-GEIGY PLC, Patent Department, Tenax**
**Road**
**Trafford Park, Manchester M17 1WT (GB)**

(54) Hirudin for the inhibition of cancer metastasis.

(57)    The invention concerns a method for the inhibition or prevention of cancer metastasis by applying hirudins and a pharmaceutical preparation therefor.

EP 0 503 829 A2

## Field of the invention

The invention relates to a method for the inhibition or prevention of tumor cell metastasis by the administration of hirudin.

## Background of the invention

The interaction of tumor cells with host compounds of hemostasis such as platelets and coagulation factors favors hematogenous metastasis. During hematogenous metastasis, tumor emboli must survive transport in the circulation, adhere to blood vessels, usually capillaries, and invade the vessel wall. Fibrin deposits and platelet aggregation around tumor emboli can protect circulating cells from mechanical trauma, facilitate their arrest in capillary beds, and protect tumor emboli from destruction by host immunity. This increased survival of emboli translates into increased production of distant visceral metastasis.

Since tumor cells possess platelet aggregation and/or procoagulant activities, the use of anticoagulants and antiplatelet agents has been advocated to decrease the chance of tumor cell survival and thus inhibit or prevent hematogenous metastasis.

Anticoagulants which have been shown to exhibit inhibition of cancer metastasis include warfarin and heparin. Furthermore, peptides which are homologous to at least a portion of the carboxy terminal 26 amino acids of hirudin have been proposed for use as anti-metastatic tumor agents (European Patent Application No. 333,356).

However, there still is a need for other effective agents capable of preventing or inhibiting tumor cell metastasis.

## Object of the invention

It is an object of the present invention to provide a method for the inhibition or prevention of cancer metastasis in a mammal.

Another object of the invention is to provide a pharmaceutical preparation useful in achieving the foregoing object.

Surprisingly, it has been found that cancer metastasis can be significantly inhibited by hirudin.

## Detailed description of the invention

The present invention is directed to a method for, and a pharmaceutical preparation useful in, the inhibition or prevention of tumor metastasis in a mammal comprising administration of hirudin in an effective amount. In particular, the invention concerns a method for, and a pharmaceutical preparation useful in, the inhibition or prevention of tumor metastasis characterised in that hirudin in an effective amount is administered to a human suffering from cancer.

Hirudin, an anticoagulant naturally occurring in leeches (Hirudo medicinalis), is not a single polypeptide species but a class of equally acting polypeptides consisting of at least four representatives designated hirudin variant 1(HV1), hirudin variant 2 (HV2) (cf. European Patent Application No. 158 564) hirudin variant 3 (PA) (cf. PCT-Application No. 86/03493] and "des-$(Val)_2$-hirudin" (cf. European Patent Application No. 158 986). The variants differ in structure from each other by a number of amino acids (especially, the N-terminal sequence of HV1 is Val-Val-Tyr, that of HV2 and of HV3 is Ile-Thr-Tyr and that of "des-$(Val)_2$-hirudin" is Thr-Tyr) but have an accumulation of hydrophobic amino acids at the N-terminus and of polar amino acids at the C-terminus, a tyrosine residue ($Tyr^{63}$) present as sulphate monoester, three disulphide bridges and the anticoagulant activity in common.

In the past few years cDNAs and synthetic genes coding for hirudin variants have been cloned and expressed in microbial hosts. Although the expression products lack the sulphate monoester group at $Tyr^{63}$ - and were therefore designated "desulphatohirudins" - they turned out to exhibit approximately the same biological activity as the natural, sulphated hirudins. Desulphatohirudin variant HV1 has been expressed in Escherichia coli (European Patent Applications No. 158 564 and 168 342) and in Saccharomyces cerevisiae (European Patent Applications No. 168 342, 200 655, 225 633, 252 854 and 341 215). Similarly, desulphatohirudin HV2 has been expressed in Escherichia coli (European Patent Applications No. 158 564) and in Saccharomyces cerevisiae [European Patent Application No. 200 655, PCT-Application No. 86/01224] and des-$(Val)_2$-desulphatohirudin has been expressed in Escherichia coli (European Patent Application No. 158 986).

According to the present invention, the term "hirudin" is intended to embrace hirudin, desulphathohirudin, a hirudin variant or a desulphatohirudin variant or a mutant thereof, respectively, described in the literature and in particular a desulphatohirudin compound or a mutant thereof obtainable from a transformed microorganism strain containing DNA which codes for a desulphatohirudin or a mutant thereof. Such desulphatohirudins are, for example, desulphatohirudin variant HV1, HV1 modified (a, b), HV2, HV2 modified (a, b, c), HV3, variants of HV3 and des(Val$_2$)-desulphatohirudin.

Preferred desulphatohirudins are those having the formula

X$_1$ Tyr Thr Asp Cys Thr Glu Ser Gly Gln Asn Leu Cys Leu Cys Glu Gly Ser Asn Val Cys

Gly Gln Gly Asn X$_2$ Cys Ile Leu Gly Ser Asp Gly Glu X$_3$ Asn Gln Cys Val Thr Gly Glu

Gly Thr Pro X$_4$ Pro Gln Ser X$_5$ Asn Asp Gly Asp Phe Glu Glu Ile Pro Glu X$_6$

(I),

in which

a) X$_1$ represents the dipeptide residue Val-Val and X$_2$, X$_3$ and X$_4$ are each Lys, X$_5$ is His and X$_6$ is the peptide residue Glu-Tyr-Leu-Gln (HV1), or

b) X$_2$ is Ile or Glu and X$_1$ and X$_3$ - X$_6$ are as defined in a) (HV1 modified a), or

c) X$_3$ is Ile or Glu and X$_1$, X$_2$ and X$_4$ - X$_6$ are as defined in a) (HV1 modified a), or

d) X$_4$ is Ile or Glu and X$_1$ - X$_3$ and X$_5$ and X$_6$ are as defined in a) (HV1 modified a), or

e) X$_5$ is Leu or Asp and X$_1$ - X$_4$ and X$_6$ are as defined in a) (HV1 modified a), or

f) X$_6$ is selected from the group consisting of Glu-Tyr, Glu-Tyr-Leu, Glu-Asp-Leu-Gln, Glu-Glu-Leu-Gln, Glu-Tyr-Lys-Arg, Glu-Asp-Lys-Arg, Glu-Lys-Leu-Gln, Ser-Phe-Arg-Tyr, Trp-Glu-Leu-Arg, Glu-Tyr-Leu-Gln-Pro and Glu-Tyr-Leu-Gln-Arg and X$_1$ - X$_6$ are as defined in a) (HV1 modified b), or.

g) in which X$_1$ represents Leu-Thr and X$_2$ - X$_6$ are as defined in a), or having the formula

Y$_1$ Tyr Thr Asp Cys Thr Glu Ser Gly Gln Asn Leu Cys Leu Cys Glu Gly Ser Asn Val Cys

Gly Lys Gly Asn Lys Cys Ile Leu Gly Ser Asn Gly Lys Gly Asn Gln Cys Val Thr Gly Glu

Gly Thr Pro Y$_2$ Pro Glu Ser His Asn Asn Gly Asp Phe Glu Glu Ile Pro Glu Glu Y$_3$ Leu

Gln

(II)

in which

a) Y$_1$ represents the N-terminal dipeptide residue Ile-Thr, Y$_2$ is Asn and Y$_3$ is Tyr (HV2), or

b) Y$_2$ is Lys, Arg or His and Y$_1$ and Y$_3$ are as defined in a) (HV2 modified a), or

c) Y$_3$ is Glu or Asp and Y$_1$ and Y$_2$ are as defined in a) (HV2 modified b), or

d) in which Y$_1$ represents the N-terminal dipeptide residue Val-Val and Y$_2$ and Y$_3$ are as defined in a) (HV2 modified c),

or having the formula

Ile Thr Tyr Thr Asp Cys Thr Glu Ser Gly Gln Asn Leu Cys Leu Cys Glu Gly Ser Asn Val

Cys Gly Lys Gly Asn Lys Cys Ile Leu Gly Ser Gln Gly Lys Asp Asn Gln Cys Val Thr Gly

Glu Gly Thr Pro Lys Pro Gln Ser His Asn Gln Gly Asp Phe Glu Pro Ile Pro Glu Asp Ala

Tyr Asp Glu

(III)

HV3 and variants of said HV3 which are characterized by a shortening of the primary structure by 1 or 2 amino acids at the N-terminus or by 18, 10, 9, 6, 4 or 2 amino acids at the C-terminus.

Particularly preferred desulphatohirudin compounds are those of formula I in which X$_1$ represents the dipeptide residue Val-Val and X$_2$, X$_3$ and X$_4$ are each Lys, X$_5$ is His and X$_6$ is the peptide residue Glu-Tyr-Leu-Gln

(Ia), or $X_1$ represents Leu-Thr and $X_2$, $X_3$ and $X_4$ are each Lys, $X_5$ is His and $X_5$ is the peptide residue Glu-Tyr-Leu-Gln (Ig) or the desulfatohirudin compound of the formula II in which $Y_1$ represents the N-terminal dipeptide residue Ile-Thr, $Y_2$ is Lys and $Y_3$ is Tyr.

The most preferred hirudin is desulfatohirudin HV1 having the formula I in which $X_1$ represents the dipeptide residue Val-Val and $X_2$, $X_3$ and $X_4$ are each Lys, $X_5$ is His and $X_5$ is the peptide residue Glu-Tyr-Leu-Gln.

The hirudins used in the present invention can be prepared synthetically, e.g. chemically or preferably by recombinant techniques, or by isolation from leeches.

According to the present invention the term "mutant" refers to proteins (muteins) exhibiting antithrombotic activity which differ from native hirudin or desulphathohirudin by simple or multiple mutations (cf. European Patent Applications No. 352 227 and No. 352 228). The DNA coding for said mutants which can be prepared by methods known in the art e.g. site-directed mutagenesis, is cloned and expressed in microbial hosts such as Escherichia coli and Saccharomyces cerevisiae.

The hirudin compounds used in the invention can be in the free form but also in the form of their salts. As they contain free amino groups in several amino acid residues, the compounds can be in the form of acid addition salts. Suitable acid addition salts are in particular pharmacologically acceptable salts with conventional therapeutically acceptable acids. Representative inorganic acids are hydrohalic acids (such as hydrochloric acid), and also sulfuric acid, phosphoric acid and pyrophosphoric acid. Representative organic acids are in particular arenesulfonic acids (such as benzenesulfonic or p-toluenesulfonic acid), or lower alkanesulfonic acids (such as methanesulfonic acid), as well as carboxylic acids such as acetic acid, lactic acid, palmitic acid, stearic acid, malic acid, tartaric acid, ascorbic acid and citric acid. As, however, the compounds used in the invention also contain free carboxyl groups in several amino acid residues, which carboxyl groups impart acidic character o the entire peptide, they can also be in the form of salts with inorganic or organic bases, e.g. sodium, potassium, calcium or magnesium salts, or also ammonium salts derived from ammonia or a pharmacologically acceptable organic nitrogen-containing base. However, as they contain at the same time free carboxyl groups and free amino groups, they can also be in the form of inner salts. Pharmacologically acceptable salts are preferred.

The present hirudins are effective in blocking or preventing malignant tumor cell induced blood coagulation and hence hematogenous metastasis of a variety of cell types in mammals, especially humans. Said cell types include, but are not limited to, fibrosarcoma, soft tissue sarcomas, chondrosarcoma, osteosarcoma, angiosarcoma, pulmonary carcinoma, breast carcinoma, carcinoma of the brain, colorectal carcinoma, renal carcinoma, prostatic carcinoma, bladder carcinoma, neoplastic plasma cell carcinoma, carcinoma of the liver, melanoma and tumors of leukocytes and progenitors, e.g. lymphomas and leukemias.

Furthermore, the invention concerns the use of hirudin for the preparation of a pharmaceutical composition for the inhibition or prevention of tumor metastasis.

According to the present invention, hirudin is administered in the form of a pharmaceutical preparation which contains at least one of the present hirudins or a pharmaceutically acceptable salt thereof, optionally together with a pharmaceutically acceptable carrier/and or adjunct.

Preferably, said preparation is a fluid which may be administered, e.g. parenterally including intraperitoneally, intramuscularly, intravenously and in particular subcutaneously. Said fluid preferably is an isotonic aqueous solution or suspension which can be prepared before use, for example from lyophilised preparations which contain the active ingredient alone or together with a pharmaceutically acceptable carrier. The pharmaceutical preparation may be sterilised and/or contain adjuvants, for example preservatives, stabilisers, wetting agents and/or emulsifiers, solubilisers, compounds for adjusting the osmolarity, e.g. mannitol, sorbitol or salts such as sodium chloride and/or a pharmaceurically acceptable buffer.

Further, according to the present invention hirudin may be administered in form of pharmaceutical liposome preparations which are produced in a manner known per se.

The present pharmaceutical preparation, which may, if desired, contain further pharmacologically valuable substances is produced in a manner known per se, for example by means of conventional dissolving or lyophilising processes, and contain from approximately 0,1 % to 100 %, especially from approximately 1 % to approximately 50 %, and in case of lyophilisates up to 100 %, of active ingredient.

The particular route of administration and the dosage will be determined by those skilled in the art taking into account particulars of the patient, the disease and the state of disease involved.

For instance, in case of continous dosing, e.g. administration by i.v. infusion, particularly by continuous infusion via mini pump, the therapeutically effective amount is in the range from approximately 0.1 to about 3 mg/kg body weight/h. The infusion can be started by a bolus of about 0.1 to about 3 mg/kg i.v. The dosage for a single i.v. bolus may range from approximately 0.1 to about 5 mg/kg body weight. Continuous dosing is envisaged e.g. in the time after initial diagnosis and prior to surgery (days to several weeks) to prevent establishment of metastasis prior to surgery.

The dosage for a single subcutaneous injection is likely to range from approximately 0.3 to approximately 10 mg/kg body weight, preferably from about 2 to about 4 mg/kg. For example, a single subcutaneous administration during operation can prevent seeding of cancer cells that are shed during the procedure.

Abbreviations

The abbreviations used hereinafter have the following meanings:
BSA        Bovine serum albumin
EMEM    Eagles' minimal essential medium
FBS        Fetal bovine serum
HBSS     Hanks' balanced salt solution
MTT        (3-(4,5-Dimethylthiazol-2-yl]-2,5-diphenyltetrazolium bromide)
IdUrd      Iododeoxyuridine
The invention is based on the following experimental methods and results:

Experimental Methods and Results

a) Reagents

Recombinant desulphatohirudin HV1 (r-hirudin), activity 11,600 antithrombin units/mg, was dissolved in sterile saline for use. MTT (Sigma Chemical Company St. Louis, MO), a stock solution was prepared by first dissolving 5 mg of MTT in 1 ml of PBS and then filtering the solution to remove particulates. The solution was stored at 4°C in the dark and used within a month.

b) Animals

Specific pathogen-free inbred female $C_{57}BL/6$ mice (Animal Production Area of the National Cancer Institute-Frederick Cancer Research Facility) were used when they were 6-10 weeks old. The care and use of the animals were in accordance with institutional guidelines.

c) Cell Line and Culture Conditions

The B16-F10 cell line selected for high lung colonization potential (Fidler, I.J. Nature (New Biol.) 242, 148-149, 1973) was maintained as adherent monolayers cultured on tissue culture plastic in Eagle's minimum essential medium (EMEM) supplemented with 10 % fetal bovine serum (FBS), sodium pyruvate, nonessential amino acids, L-glutamine, and vitamin solution (GIBCO Laboratories, Grand Island, NY) at 37°C in a humidified incubator with 5 % $CO_2$ and 95 % air. The cell line was free of mycoplasma and the following pathogenic mouse viruses: reovirus type 3, murine pneumonia virus, K virus, Theiler's virus, Sendai virus, murine minute virus, murine adenovirus, murine hepatitis virus, lymphocytic choriomeningitis virus, ectromelia virus, and lactate dehydrogenase virus (assayed by Microbiological Associates, Wakersville, MD). To ensure reproducibility of in vivo and in vitro assays, the cultures were used within 4 weeks after recovery from frozen stocks.

d) Preparation of Tumor Cell Suspensions

For in vivo injection, tumor cells were harvested from subconfluent cultures (50 -80 % confluency) by overlaying with 0.25 % trypsin and 0.02 % EDTA. After 1 min, the flasks were sharply tapped to dislodge cell aggregates, which were then gently pipetted into medium containing 10 % FBS. For i.v. injections, the cells were resuspended in $Ca^{2+}$- and $Mg^{2+}$-free Hanks' balanced salt solution (HBSS). For in vitro assays of clotting, tumor cells were harvested by mechanical dissociation with a rubber policeman in the absence of proteases after the monolayers were washed twice with $Ca^{2+}$- and $Mg^{2+}$-free HBSS and cell concentration adjusted to 1 x $10^6$ ml. Only single-cell suspensions of greater than 90 % viability as determined by trypan blue exclusion were used in the in vivo and in vitro experiments.

e) Determination of Direct in Vitro Effects of r-hirudin on Cell Growth

Tumor cells were seeded into flat-bottom 96-well plates in triplicate at the density of 2 x $10^3$ cells per 38 $mm^2$ well in 0.1 ml of supplemented EMEM containing 10 % FBS. r-Hirudin in 0.1 ml of supplemented EMEM was added at final concentrations of 0.001-10 μM per 0.2 ml volume/well. After 96 h incubation, cell proliferation was determined by the MTT assay using 40 μl of MTT stock solution incubated with the cells at 37°C in 5 %

$CO_2$ for 2 - 4 h. The cells were then washed and lysed in DMSO, and the conversion by metabolically viable cells of MTT to formazan was monitored by a Titertek Multiskan 96-well microtiter plate reader at 570 nm (Flow Laboratories, Helsinki, Finland) (Fan, D. Cancer Communic. 1, 117-125, 1989). The results were calculated as follows:

$$\% \text{ cytostasis } = [1 - (A/B)] \times 100,$$

where A = absorbance of r-hirudin-treated cells, and B = absorbance of untreated control cells.

### f) Preparation of Citrated Human and Mouse Plasma

Humam blood was obtained from healthy donors who had not taken anticoagulants for at least 10 days prior to the assay. Trisodium citrate (final concentration, 0.38 % w/v) was used as an anticoagulant. Mouse blood was obtained by cardiac puncture of anesthetized $C_{57}BL/6$ mice and immediately mixed with trisodium citrate. Platelet-free plasma was prepared by centrifugation of the citrated blood at 2000 x g for 20 min at 4°C.

### g) Clotting Assays

Procoagulant activity of B16-F10 cells was measured by the single stage recalcification time (Pearlstein, E. et al. Cancer Res. 41, 4535-4539, 1981; Esumi, N. et al. Cancer Res. 47, 2129-2135, 1987). One hundred microliters of tumor cell suspension or HBSS serving as a control were added to 100 μl of plasma prewarmed for 2 min at 37°C in a glass tube, and the mixture was incubated for another minute. The reaction was initiated by adding 100 μl prewarmed 25 mM $CaCl_2$, and the clotting time was measured in seconds. Tissue thromboplastin from rabbit brain (Sigma Chemical Company) was used as a control for production of thrombin. The inhibitory effect of r-hirudin on this coagulation system was determined by adding 10 μl of r-hirudin solution at a final concentration ranging from 0.001 to 10 μM before the addition of $CaCl_2$. Prolongation of clotting time was then measured. All assays were carried out in triplicate.

### h) Production of Experimental Lung Metastasis

B16-F10 cells at the concentration of 25,000 cells/0.2 ml HBSS/mouse were injected into the lateral tail vein of unanesthetized $C_{57}BL/6$ mice. The mice were monitored daily and killed 3 weeks later. The lungs were removed, washed in water, and fixed in Bouin's solution. The number of lung nodules was determined under a dissecting microscope.

To study the extent and the duration of effect of r-hirudin on experimental metastasis, r-hirudin solution was injected s.c. at the dose of 200 μg/0.2 ml PBS/mouse (10 mg/kg mouse weight) at various times before or after i.v. injection of B16-F10 cells. In other experiments, different doses of r-hirudin, ranging from 3 to 400 μg/mouse (0.15-20 mg/kg mouse weight) were administered s.c. 15 min before the i.v. injection of 25,000 B16-F10 cells/mouse.

To rule out the possibility that r-hirudin mediates a direct antitumor effect, B16-F10 cells were incubated at room temperature with r-hirudin in PBS at concentrations of 0.1 to 10 μM for 30 min. The cells were then washed twice with PBS, resuspended in $Ca^{2+}$- and $Mg^{2+}$-free HBSS, and injected i.v. into mice (n=5), which were killed 3 weeks later. The number of experimental lung metastases was determined under a dissecting microscope.

### i) In Vitro Labeling of Cells with [125I]Iododeoxyuridine [125I]IdUrd

Tumor cells were seeded into 150 $cm^2$ tissue culture flasks at the density of 4 x $10^6$ cells/flask. Twenty-four hours later, 0.3 μCi/ml of [125I]IdUrd (New England Nuclear, Boston, MA; specific activity 2000 mCi/mM) was added to the supplemented EMEM. Twentyfour hours later, the monolayers were rinsed twice with excess $Ca^{2+}$- and $Mg^{2+}$-free HBSS to remove nonbound radioiodine. The cells were then harvested with 0.25 % trypsin- 0.02 % EDTA solution as described above and suspended in medium. The cell suspension was washed and resuspended in $Ca^{2+}$- and $Mg^{2+}$-free HBSS at a concentration of 1 x $10^5$ cells/0.2 ml HBSS, the inoculum volume per mouse.

### j) Distribution and Fate of [125I]IdUrd-labeled Cells after i.v. Injection

Labeled B16-F10 cells (1 x $10^5$/mouse) were injected i.v. into unanesthetized $C_{57}BL/6$ mice. To study the effect of r-hirudin on the initial organ distribution of the tumor cells, we injected $C_{57}BW6$ mice s.c. with PBS (group A) or r-hirudin in PBS (200 μg/mouse; 10 mg/kg mouse weight) 15 min before (group B) or 60 min after (group C) the i.v. inoculation of tumor cells. At various intervals after i.v. injection, groups of mice (n=5) weie killed. Lung, liver, kidneys, and spleen of each mouse were removed and placed in test tubes containing 70 % ethanol. The ethanol was replaced daily for 3 days to remove all soluble $^{125}$I released from dead cells (Fidler, I.J. J. Natl. Cancer Inst. 45, 773-782, 1970). In addition, 0.2 ml of blood from each mouse was collected and placed in a test tube. The radioactivity in all samples was determined in a gamma counter (TM Analytic, Elk Grove Village, IL). Triplicate tubes containing the inoculum dose were retained, and the radioactivity was determined at the same time as the sample organs.

In another set of experiments, the organs collected from each mouse were washed once in PBS and placed in test tubes, and the radioactivity was measured immediately rather than after 3 days of ethanol wash. The mean count in organs from each group of mice (n=5) at each time point was expressed as the percentage of input counts. All measurements were corrected for radioactive decay of input cells.

k) Preparation for Histolopy

C$_{57}$BL/6 mice were injected s.c. with r-hirudin (200 μg/mouse) or with PBS, and 15 min later, 25,000 B16-F10 cells/0.2 ml HBSS were injected i.v. into groups of mice (n=3). The mice were killed 15 min, 1 h, 4 h, 24 h, and 3 weeks after tumor cell inoculation and necropsied, and lungs were fixed in 10 % buffered formalin. Sections 4-μm thick from each lung were stained with hematoxylin and eosin.

Procoagulant Activity of B16 - F10 Cells and Inhibition by r-Hirudin (In Vitro Assay)

B16-F10 cells have been previously shown to have a procoagulant activity through direct activation of Factor X (VanDeWater, L. et al., Cancer Res. 45, 5521 -5525, 1985). In our study, B16-F10 cells significantly shortened the single stage recalcification time in mouse plasma, but exhibited low procoagulant activity in human plasma, suggesting that this activity was species-specific. In contrast, tissue thromboplastin used as a positive control exibited a significant shortening of the clotting time in both mouse and human plasma. r-Hirudin significantly inhibited clotting induced by tissue thromboplastin or by B16-F10 cells in both mouse and human plasma, indicating that the potent thrombininhibiting activity of r-hirudin was not species-specific (data not shown).

Effect of r-Hirudin on Formation of Experimental B16-F10 Lung Metastasis

The production of experimental lung metastasis by B16-F10 cells was significantly inhibited by the s.c. injection of r-hirudin, but the extent of this inhibition was dependent on the time of administration (Table 1). Significant inhibition of B16-F10 experimental lung metastasis was obtained with s.c. administration of r-hirudin at time points ranging from 120 min before to 60 min after tumor cell inoculation, with the most dramatic effects found in mice given r-hirudin 15 min or 2 min before i.v. injection of tumor cells (P<0.001 ). In another set of experiments, r-hirudin injected s.c. at times ranging from 240 min before or 60, 120, and 240 min after i.v. tumor cell injection did not inhibit experimental metastasis (Table 1). Extrapulmonary tumor colonies were not found in any of the mice treated with PBS or with r-hirudin.

Table 1. Inhibitory Effects of r-Hirudin on Experimental Lung
Metastasis Produced by B16-F10 Melanoma Cells

| Time of r-hirudin s.c. administration (min)[a] | Number of lung nodules median (range)[b] | |
|---|---|---|
| | Expt. 1 | Expt. 2 |
| -240 | N.D.[c] | 40 (18-68) |
| -120 | 28 (14-39)[e] | 21 (2-42)[e] |
| -60 | 12 (2-24)[d] | 15 (3-29)[d] |
| -30 | 9 (3-49)[d] | N.D.[c] |
| -15 | 5 (1-9)[d] | N.D.[c] |
| -2 | 4 (2-9)[d] | 1 (0-4)[d] |
| +15 | 12 (6-24)[d] | N.D.[c] |
| +30 | 24 (1-47)[e] | N.D.[c] |
| +60 | 51 (27-62)[e] | 30 (18-38) |
| +120 | 80 (34-106) | 35 (17-50) |
| +240 | N.D. | 41 (20-59) |
| PBS control | 83 (58-133) | 49 (32-67) |

[a]r-Hirudin was administered s.c. at a dose of 10 mg/kg mouse
weight (200 ug/mouse) before (-240, -120, -60, -30, -15,
-2 min) or after (+15, +30, +60, +120, +240 min) i.v.
injection of tumor cells.

[b]B16-F10 cells (25,000/0.2 ml HBSS) were injected i.v. into
syngeneic mice. The number of experimental lung metastases
was determined 3 weeks later. Each treatment group
consisted of 10 mice.

[c]N.D., not done.

[d]significantly different from control. p<0.001, Mann Whitney
U-test (2-tailed).

[e]significantly different from control. p<0.01, Mann Whitney
U-test (2-tailed).

To investigate the dose relationship of r-hirudin to inhibition of experimental B16-F10 melanoma lung metastasis, we administered different doses of r-hirudin s.c. 15 min before the i.v. injection of tumor cells. The data in Table 2 show that r-hirudin inhibited experimental metastasis in a dose-dependent manner and that the dose of 10 mg/kg mouse weight used in most of our experiments achieved near maximal effects.

Table 2. Dose-dependent Inhibitory Activity of r-Hirudin on
Formation of Experimental B16-F10 Lung Metastasis

| Dose of r-hirudin (mg/kg mouse weight)[a] | Number of lung nodules median (range)[b] |
|---|---|
| 20 | 2 (1-3)[c] |
| 10 | 5 (1-6)[c] |
| 5 | 11 (1-16)[c] |
| 2.5 | 10 (6-19)[c] |
| 1.25 | 15 (7-20)[c] |
| 0.63 | 24 (14-32)[d] |
| 0.31 | 26 (18-42) |
| 0.16 | 28 (20-40) |
| PBS control | 49 (32-67) |

[a] r-Hirudin was administered s.c 15 min before tumor cell inoculation.

[b] B16-F10 cells (25,000/0.2 ml HBSS) were injected i.v. into syngeneic mice. The number of experimental lung metastases was determined 3 weeks later. Each treatment group consisted of 10 mice.

[c] $P<0.001$. Mann Whitney U-test (2-tailed).

[d] $P<0.01$. Mann Whitney U-test (2-tailed).

### Distribution and Fate of [125I]IdUrd-labeled B16-F10 Cells after i.v. Injection

The organ distribution and fate of [125I]IdUrd-labeled tumor cells were determined from 10 min to 24 h after i.v. injection into 3 treatment groups of mice: mice treated s.c. with PBS (group A), mice treated s.c. with r-hirudin 15 min before tumor cell injection (group B), and mice treated s.c. with r-hirudin 60 min after tumor cell injection (group C). The most impressive change in the distribution of labeled cells were observed in the lungs (Table 3). A significant decrease in initial tumor cell arrest in the lung was found in mice treated with r-hirudin 15 min before tumor cell injection (group B). In control mice, we found 79.6 % of the cells in the lungs, whereas in treated mice, it was 10.7 % (P<0.01). These results were obtained subsequent to 3 daily washes with 70 % ethanol (Gasic, G.J. Cancer Metastasis Rev. 3, 99-114, 1984). If organs were only washed once in PBS and then immediately monitored for radioactivity, the difference was not found. Under these conditions, cell arrest in the lungs of mice from groups A and B was 92.8 % and 85.8 %, respecrively. The exact reason for this discrepancy is unclear. It could be due to alcohol displacement of unarrested tumor cells in the lungs of r-hirudin-treated mice.

No significant differences in cell survival in the lungs of the 3 groups were found at 60 min; however, by 120 min, a clear difference emerged. By 24 h, 1.6 % of injected B16-F10 cells survived in the lungs of PBS-treated mice, whereas in mice treated with r-hirudin 15 min before or 60 min after tumor cell injection, the survival was 0.06 % and 0.46 %, respectively (Table 3). The differences in the survival of [$^{125}$I]IdUrd-labeled B16 cells in the lungs of mice directly correlated with the number of tumor colonies found 3 weeks later (Tables 1, 2).

The distribution of radiolabeled tumor cells in the liver differed between mice in groups A and B. As shown in Table 3, there was no difference in the radioactivity at 10 min and 60 min. By 120 min, however, a higher percentage of cells was found in the liver of mice treated with r-hirudin as compared to controls. This difference in proportion probably reflects the absolute decrease in viable cells arrested in the lung or present in the blood. In r-hirudin-treated mice (group B), about 10 % of injected cells were found in the blood at 120 min after tumor cell injection. Even after 240 min, 7.6 % of the cells were still detected in blood, suggesting that many tumor cells in mice treated with r-hirudin did not arrest in the microcirculation of the lung and did not adhere to vascular endothelium. By 24 h, the differences in the level of radioactivity in the liver and blood disappeared, and the percentage of surviving tumor cells decreased to less than 1 % of the original inoculum. The distribution of radiolabeled cells in the liver and blood in group C did not differ from that of PBS-treated mice. The distribution of radiolabeled cells to the spleen and the kidneys was unremarkable and did not differ among the 3 treatment groups (Table 3).

EP 0 503 829 A2

Table 3. Distribution and Fate of [$^{125}$I]IdUrd-labeled B16-F10 Cells after I.V. Injection

| | Lungs | | | Liver | | | Spleen | | | Kidneys | | | Blood[d] | | |
|---|---|---|---|---|---|---|---|---|---|---|---|---|---|---|---|
| | | | | | | | % of input radioactivity[c] | | | | | | | | |
| Time[a] | PBS | H (-15)[b] | H (+60)[b] | PBS | H (-15) | H (+60) | PBS | H (-15) | H (+60) | PBS | H (-15) | H (+60) | PBS | H (-15) | H (+60) |
| 10 min | 79.6 | 10.7 | - | 1.8 | 1.6 | - | 0.06 | 0.10 | - | 0.16 | 0.01 | - | 3.6 | 2.6 | - |
| | (92.8)[e] | (85.8)[e] | | (4.8)[e] | (8.9)[e] | | | | | | | | | | |
| 60 min | 58.5 | 67.0 | - | 0.7 | 1.3 | - | 0.16 | 0.06 | - | 0 | 0.13 | - | 2.8 | 9.2 | - |
| 120 min | 50.2 | 32.7 | 58.4 | 1.0 | 3.4 | 1.5 | 0.10 | 0.20 | 0.32 | 0.10 | 0.12 | 0.05 | 4.0 | 10.4 | 5.8 |
| 240 min | 26.6 | 4.3 | 9.3 | 0.7 | 0.3 | 0.4 | 0.20 | 0.02 | 0.11 | 0.04 | 0.17 | 0.07 | 5.0 | 7.6 | 5.4 |
| 24 h | 1.6 | 0.06 | 0.46 | 0.3 | 0.07 | 0.01 | 0.15 | 0.06 | 0 | 0.01 | 0.05 | 0.15 | 0 | 0 | 0.12 |

[a] [$^{125}$I]IdUrd-labeled viable tumor cells were injected i.v. (1 x 10$^5$/mouse) and groups of 5 mice were killed at each time point.

[b] r-Hirudin was administered s.c. at a dose of 10 mg/kg mouse weight (200 μg/mouse) 15 min before [H (-15)] or 60 min after [H (+60)] tumor cell inoculation.

[c] Percentage of radioactivity (mean of 5 mice) was determined after 3 daily washes of the organ samples with 70 % ethanol. The standard deviations were less than 10 % in all samples.

[d] For blood samples, radioactivity in 0.2 ml of blood per mouse was measured and expressed as a percentage of input in total blood volume which was estimated to be 2.0 ml.

[e] The results obtained directly without 3 day washing with ethanol at 10 min are indicated in parenthesis.

The following example is for illustrative purpose only and is not to be construed as limiting this invention in any manner.

Example 1: Pharmaceutical composition containing desulphatohirudin HV1 for parenteral administration

A solution containing desulphatohirudin HV1 is dialysed against a 0.9 % NaCl solution. The concentration of the solution is then adjusted by diluting with the same NaCl solution to 0.2 mg/ml or 2 mg/ml. These solutions are sterilized by ultrafiltration (membranes with 0.22 $\mu$m pores).

The sterilized solution can be used directly, for example for intravenous administration.

**Claims**

1. The use of hirudin for the preparation of a pharmaceutical composition for the inhibition or prevention of tumor metastasis.

2. The use as claimed in claim 1 in which said hirudin is a desulphatohirudin variant or a mutant thereof.

3. The use as claimed in claim 1 or 2 in which said hirudin is desulphatohirudin HV1.

4. The use as claimed in any preceding claim in which said pharmaceutical composition is for parenteral administration.

5. The use as claimed in any preceding claim in which said pharmaceutical composition is for administration to a mammal.

6. The use as claimed in claim 5 in which said mammal is a human being.

7. A method for the inhibition or prevention of tumor metastasis in a mammal which comprises administering to said mammal an effective amount of hirudin.

8. A pharmaceutical composition containing hirudin for the inhibition or prevention of tumor metastasis in a mammal.